(19) Europäisches Patentamt European Patent Office Office européen des brevets

(11) **EP 3 978 001 B1**

(12) **EUROPEAN PATENT SPECIFICATION**

(45) Date of publication and mention of the grant of the patent:
**13.11.2024 Bulletin 2024/46**

(21) Application number: **20813731.5**

(22) Date of filing: **07.05.2020**

(51) International Patent Classification (IPC):
***A61K 31/593*** *(2006.01)*    ***A61K 31/138*** *(2006.01)*
***A61P 17/00*** *(2006.01)*

(52) Cooperative Patent Classification (CPC):
(C-Sets available)
**A61P 17/00; A61K 31/138; A61K 31/593**    (Cont.)

(86) International application number:
**PCT/CN2020/088902**

(87) International publication number:
**WO 2020/238570 (03.12.2020 Gazette 2020/49)**

(54) **COMPOSITION CONTAINING FLUOXETINE AND VITAMIN DOR ITS DERIVATIVES, AND APPLICATION THEREOF**

ZUSAMMENSETZUNG MIT FLUOXETIN UND VITAMIN D ODER DESSEN DERIVATEN UND ANWENDUNG DAVON

COMPOSITION CONTENANT DE LA FLUOXÉTINE ET DE LA VITAMINE D OU SES DÉRIVÉS, ET SON APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2019 CN 201910450610**

(43) Date of publication of application:
**06.04.2022 Bulletin 2022/14**

(73) Proprietor: **Nanjing Ruiying Runze Biopharmaceutical Technology Co., Inc.**
**Jiangsu 211100 (CN)**

(72) Inventors:
• **SHANG, Jing**
**Nanjing City, Jiangsu Province, 211198 (CN)**
• **ZHONG, Hui**
**Nanjing City, Jiangsu Province, 211198 (CN)**
• **PENG, Jixian**
**Heze, Shandong 274000 (CN)**
• **YUE, Yunyun**
**Nanjing, Jiangsu 211100 (CN)**
• **ZHOU, Liangliang**
**Nanjing, Jiangsu 211100 (CN)**
• **ZHU, Junyi**
**Nanjing, Jiangsu 211100 (CN)**

(74) Representative: **Patentanwälte**
**Ruff, Wilhelm, Beier, Dauster & Partner mbB**
**Kronenstraße 30**
**70174 Stuttgart (DE)**

(56) References cited:
**CN-A- 102 429 894    CN-A- 106 667 903**
**CN-A- 108 969 512    US-B2- 9 833 424**

• **ZHOU LIANGLIANG ET AL: "The different roles of 5-HT1A/2A receptors in fluoxetine ameliorated pigmentation of C57BL/6 mouse skin in response to stress", JOURNAL OF DERMATOLOGICAL SCIENCE, ELSEVIER, AMSTERDAM, NL, vol. 92, no. 3, 25 October 2018 (2018-10-25), pages 222 - 229, XP085563291, ISSN: 0923-1811, DOI: 10.1016/J.JDERMSCI.2018.10.002**
• **PARSAD DAVINDER ET AL: "Topical vitamin D 3 analogues in the treatment of vitiligo", PIGMENT CELL & MELANOMA RESEARCH, vol. 22, no. 4, 1 August 2009 (2009-08-01), United States, Denmark, pages 487 - 488, XP093046125, ISSN: 1755-1471, DOI: 10.1111/j.1755-148X.2009.00579.x**

EP 3 978 001 B1

- AMEEN M ET AL: "Topical calcipotriol as monotherapy and in combination with psoralen plus ultraviolet A in the treatment of vitiligo", BRITISH JOURNAL OF DERMATOLOGY, JOHN WILEY, HOBOKEN, USA, vol. 145, no. 3, 18 July 2008 (2008-07-18), pages 476 - 479, XP071117587, ISSN: 0007-0963, DOI: 10.1111/J.1365-2133.2001.04381.X
- KHORAMINYA, NAYEREH ET AL.,: "Therapeutic effects of vitamin D as adjunctive therapy to fluoxetine in patients with major depressive disorder,", AUSTRALIAN AND NEW ZEALAND JOURNAL OF PSYCHIATRY,, vol. 47, no. 3, 31 March 2013 (2013-03-31), XP055762827, DOI: 20200616102213X
- MIN YI-GUO, HUANG YONG-HUA: "The Molecular Biology Mechanism and Application status of Vitamin D3 Analogs for The Treatment of Vitiligo", MEDICAL INNOVATION OF CHINA, vol. 9, no. 24, 25 August 2012 (2012-08-25), pages 162 - 164, XP055878434, ISSN: 1674-4985, DOI: 10.3969/j.issn.1674-4985.2012.24.103
- XU QIAN-XI , DU JUAN , HE PEI-YING ,ZHANG JIAN-ZHONG ,ZHU TIE-JUN: "Effects of 1$\alpha$,25-dihydroxyvitamin D3 and UVB on cell proliferation and melanin synthesis of cultured human melanocyte", JOURNAL OF PEKING UNIVERSITY(HEALTH SCIENCES), vol. 36, no. 5, 18 October 2004 (2004-10-18), pages 483 - 486, XP055878435, ISSN: 1671-167x, DOI: 10.19723/j.issn.1671-167x.2004.05.010

(52) Cooperative Patent Classification (CPC): (Cont.)

C-Sets
A61K 31/138, A61K 2300/00;
A61K 31/593, A61K 2300/00

## Description

### TECHNICAL FIELD

The present invention relates to a drug for treating and/or relieving depigmentation disorders. More specifically, it relates to a composition comprising fluoxetine and $VD_3$ or its derivatives, its use in preparing a drug for treating and/or relieving depigmentation disorders, and a pharmaceutical preparation comprising the composition.

### BACKGROUND

Depigmentation disorders are common skin diseases with local depigmentation of skin as the main clinical symptoms, such as vitiligo, white hair, pityriasis alba, nevus anemicus and so on, among which vitiligo and white hair are the most common. Vitiligo is a kind of depigmentation disorder of the skin which is characterized by local or generalized depigmented white patches on the skin and mainly occurs in children and adolescents. White hair refers the whole or part of the hair becomes white, which can be divided into two types: congenital white hair and acquired hair. Congenital white hair often has a family history; while acquired white hair includes canities senilis and premature canities suffered by adolescents and middle-aged people. The main manifestation of depigmentation disorders is the absence of melanocytes or melanogenesis in skin or hair. Melanin in skin is mainly distributed in melanocytes in the basal layer of skin epidermis which originates from embryonic ectoderm, and is mainly composed of keratinocytes (accounting for about 80%-90% of epidermal cells), non-keratinocytes (also referred as dendritic cells which are mainly melanocytes; accounting for about 2%-3% of epidermal cells), Langerhans cells and Merkel cells. Melanocytes are mainly distributed in the basal layer of skin, iris of eyes and hair follicles, especially in epidermis. The total number of melanocytes in the basal layer of epidermis is 2 billion, and melanocytes in epidermis are the key elements to determine skin color and prevent damage from ultraviolet radiation. Therefore, when the epidermal melanocytes are damaged or the function of melanogenesis is abnormal, the skin may have pigment disorder or other diseases, such as vitiligo, white hair, nevus or melanoma [1].

Chinese patent ZL201110403173.4 and CA2,877,423A disclose use of fluoxetine in treating depigmentation disorders, which mainly describe that fluoxetine can promote synthesis of B16F10 cells and the melanin of normal human skin melanocytes, and promote the up-regulation of the expression of melanogenesis related proteins. At the same time, oral administration of fluoxetine can improve the expression of skin pigmentation synthesis related proteins of melanogenesis in C57BL/6 mice. It is the first time to apply fluoxetine to treat depigmentation disorders. It has been reported that oral administration of fluoxetine at 20 mg/kg can promote skin coloring in normal mice, which may be related to its regulation of the expression of 5-HT1A receptor in skin [2]. At the same time, oral administration of fluoxetine at 2.6 mg/kg can improve skin discoloration of unhaired back of C57BL/6 mice induced by chronic unpredictable stress and chronic restraint stress by regulating 5-HT1A receptor and 5-HT2A receptor in skin [3]. U.S. Pat. No. US9,833,424B2 discloses that the external preparation prepared by fluoxetine can be used for treating vitiligo at a dose of 0.1% g/g. The above patents and literatures show that fluoxetine can be used to treat depigmentation disorders in a certain dosage range (2.6 mg/kg-20 mg/kg).

Clinical studies have found that the lower levels of vitamin D and its metabolite 1,25-dihydroxyvitamin $D_3$ (1,25-$(OH)_2D_3$) in vitiligo patients compared with normal people may be related to the onset of vitiligo [4]. Vitiligo patients have high expression of autoantibody Ig, which can destroy melanocytes and form white patches through complement lysis and cytotoxicity. 1,25-$(OH)_2D_3$ can reduce the damaging effect of autoantibodies on melanocytes by inhibiting the production of autoantibodies [5]. At the same time, calcipotriol, a derivative of $VD_3$, can affect the dendrites of melanocytes by regulating the autophagy process of melanocytes, thus reducing the damage of melanocytes caused by oxidative stress [6]. The research results suggest that the skin microenvironment can affect the function of melanocytes, and calcipotriol can improve the living environment of melanocytes by regulating the function of immune cells, thus avoiding the apoptosis of melanocytes and the absence of melanin synthesis function. Although the consensus of diagnosis and treatment of vitiligo (2018 edition) suggests that $VD_3$ derivatives can be used to treat vitiligo [7,8], among which tacalcitol and calcipotriol are the representative drugs, but their clinical indication is psoriasis. Calcipotriol used in monotherapy or in combination with psoralen plus ultraviolet A was effective and well tolerated in the treatment of vitiligo [14,15]. Tacalcitol has been shown to be effective in the treatment of vitiligo when combined with PUVA and NBUVB [14]. Calcipotriol was first marketed by LEO Pharma in 1991 as a drug for the local treatment of psoriasis. In 2002, LEO Pharma introduced carbotriol liniment (trade name: Darius; Specifications: 50 $\mu$g/ml (about 50 $\mu$g/g) and calcipotriol ointment (Specification: 50 $\mu$g/g) into Chinese market.

It is reported that calcipotriol ($10^{-9}$-$10^{-5}$ mol/L), that is, about $4.13 \times 10^{-4}$-$4.13 \mu$g/g, can promote melanogenesis of normal melanocytes [9]. However, it is reported clinically that calcipotriol ointment (50 $\mu$g/g) is easy to cause side effects such as rash and flushing on the face during the treatment of psoriasis, and at the same time, there may be a risk of increasing blood calcium content [10]. And long term oral administration of fluoxetine (20 mg/ day) may cause

3

side effects, such as insomnia, lethargy, suicide, neurological disorders and so on [11]. Chinese patent publication No. 107375428A discloses a method for treating vitiligo by combining fluoxetine with traditional Chinese medicine.

After long-term and in-depth research, the inventors of the present invention have found that when fluoxetine is used in combination with $VD_3$ derivatives for treat vitiligo, a better synergistic effect of treating vitiligo can be obtained, and at the same time, the dose of each drug can be reduced so as to avoid the side effects in the clinical use of each single drug.

Fluoxetine in combination with $VD_3$ derivatives can improve melanogenesis. Compared with $VD_3$ derivatives or fluoxetine alone, fluoxetine in combination with $VD_3$ derivatives can not only improve the curative effect, but also reduce the dosage of $VD_3$ derivatives or fluoxetine and the occurrence of toxic and side effects.

## SUMMARY OF THE INVENTION

The inventors of the present invention have found that fluoxetine in combination with $VD_3$ or its derivatives can effectively reduce the toxicity of both components, fluoxetine in combination with $VD_3$ or its derivatives can promote melanogenesis in a dose-dependent manner, and can promote the increase of tyrosinase activity in a dose-dependent manner, so they can be used for treating or relieving depigmentation disorders caused by the decrease of melanogenesis. The decrease of vitamin $D_3$ content in vitiligo patients causes the decrease of 5-HT level. Fluoxetine is a 5-HT reuptake inhibitor. Fluoxetine in combination with $VD_3$ or its derivatives can restore the 5-HT level to normal level. The inventors have found that the combined use of fluoxetine (0.01-10 mg/g) and calcipotriol (0.5-50 $\mu$g/g) in a certain dosage range can produce synergistic effect, which has better effect on melanogenesis than that when using fluoxetine and calcipotriol alone, and also has better effect than that when using fluoxetine and calcipotriol separately. The combined use of calcipotriol and fluoxetine in the clinical treatment of depigmentation disorders can not only improve the efficacy, but also reduce the dosage and side effects on the basis of ensuring the efficacy.

Therefore, it is an object of the present invention to provide a preparation comprising a composition comprising fluoxetine and vitamin D3 or derivatives of vitamin D3, wherein vitamin D3 or its derivatives is one or more selected from the group consisting of vitamin D3, calcipotriol, tacalcitol and calcitriol, and the preparation is a cream, an ointment, a gel or a paste, the preparation for use in the treatment and/or alleviation of depigmentation disorder, and use in treating white hair.

The technical solution of the present invention is as follows.

According to one aspect of the present invention, it provides a composition comprising fluoxetine and $VD_3$ or its derivatives, wherein vitamin D3 or its derivatives is one or more selected from the group consisting of vitamin D3, calcipotriol, tacalcitol and calcitriol, which is used for treating and/or alleviating depigmentation disorders.

Preferably, the weight ratio of fluoxetine to $VD_3$ or its derivatives aforementioned is 10 or more, preferably 20 or more.

Preferably, the weight ratio of fluoxetine to $VD_3$ or its derivatives aforementioned is 500 or less, preferably 400 or less, more preferably 200 or less.

Preferably, fluoxetine is selected from the group consisting of fluoxetine racemate, R-fluoxetine and their pharmaceutically acceptable salts.

$VD_3$ or its derivatives is one or more selected from the group consisting of $VD_3$, calcipotriol, calcipotriol, rocaltrol and tacalcitol.

The composition may further comprise pharmaceutically acceptable excipients as long as the excipients do not impair the object of the present invention, and exemplified examples thereof include, but are not limited to, emulsifiers, antioxidants, wetting agents, humectants, surfactants, polymer compounds, pH regulators, metal ion complexing agents, propellants, solvents, liquid oils and fats, and so on.

When the composition comprises excipients, the content of fluoxetine is 0.01 to 10 mg per g of the composition, preferably 0.01 to 5 mg per g of the composition, more preferably 0.1 to 1 mg per g of the composition.

Preferably, the content of $VD_3$ or its derivatives is 0.005 to 500 $\mu$g per g of the composition, preferably 0.5 to 250 $\mu$g per g of the composition, more preferably 5 to 50 $\mu$g per g of the composition.

According to another aspect of the present invention, it provides use of the above composition in preparing drugs for treating and/or alleviating depigmentation disorders.

The depigmentation disorders are diseases caused by the decrease of melanogenesis, which is selected from the group consisting of vitiligo, pityriasis alba, nevus anemicus, albinism and so on.

According to another aspect of the present invention, it provides use of the composition in treating white hair.

According to yet another aspect of the present invention, it provides a preparation comprising the composition according to the present invention.

The preparation is selected from the group consisting of cream, ointment, gel, aerosol, spray, powder mist, solution, aromic waters, tincture, spiritus, glycerite, sol, suspension, emulsion, liniment, coating agent, paste, lotion, liniment and tincture, etc.

According to another aspect of the present invention, it provides a method for treating depigmentation disorders, which comprises: coating the composition according to the present invention or a preparation made of the composition on the skin by topical administration.

Preferably, in the composition or the preparation made of the composition, the concentration of fluoxetine is 0.01 to 10 mg per g of the composition, preferably 0.01 to 5 mg per g of the composition, more preferably 0.1 to 1 mg per g of the composition.

The depigmentation disorders are diseases caused by the decrease of melanogenesis, which are selected from the group consisting of vitiligo, pityriasis alba, nevus anemicus, albinism and so on.

R-fluoxetine and $VD_3$ or its derivatives in the composition according to the present invention can cooperate with each other to reduce each other's toxicity, promote melanogenesis and increase tyrosinase activity. Therefore, depigmentation disorders can be effectively treated and/or alleviated.

**BRIEF DESCRIPTION TO THE DRAWING**

Fig. 1 is a flow chart showing some experiments of Example 1, Example 2, Example 3, Example 4, Example 5, Example 6 and Example 7.

Fig. 2 is a graph showing the effect of fluoxetine, $VD_3$, calcipotriol and tacalcitol when used alone on the survival rate of zebrafish in Example 1.

Fig. 3 is a graph showing the effect of fluoxetine when used in combination with different concentrations of $VD_3$ on survival rate of zebrafish in part 2.1 of Example 2.

Fig. 4 is a graph showing the effect of fluoxetine when used in combination with different concentrations of calcipotriol on survival rate of zebrafish in part 2.2 of Example 2.

Fig. 5 is a graph showing the effect of fluoxetine when used in combination with different concentrations of tacalcitol on survival rate of zebrafish in part 2.3 of Example 2.

Fig. 6 is a graph showing the effect of $VD_3$ when used in combination with different concentrations of fluoxetine on survival rate of zebrafish in part 2.4 of Example 2.

Fig. 7 is a graph showing the effect of calcipotriol when used in combination with different concentrations of fluoxetineon on survival rate of zebrafish in part 2.5 of Example 2.

Fig. 8 is a graph showing the effect of tacalcitol when used in combination with different concentrations of fluoxetine on survival rate of zebrafish in part 2.6 of Example 2.

Fig. 9 is a graph showing the effect of fluoxetine when used alone on the melanin content of zebrafish in part 3.1 of Example 3.

Fig. 10 is a fitting diagram showing the dose-effect relationship of fluoxetine when used alone in part 3.1 of Example 3.

Fig. 11 is a graph showing the effect of fluoxetine when used alone on tyrosinase activity of zebrafish in part 3.2 of Example 3.

Fig. 12 is a graph showing the effect of $VD_3$ when used alone on the melanin content of zebrafish in part 4.1 of Example 4.

Fig. 13 is a fitting diagram showing the dose-effect relationship of $VD_3$ when used alone in part 4.1 of Example 4.

Fig. 14 is a graph showing the effect of calcipotriol when used alone on tyrosinase activity of zebrafish in part 4.2 of Example 4.

Fig. 15 is a graph showing the effect of calcipotriol when used alone on the melanin content of zebrafish in part 5.1 of Example 5.

Fig. 16 is a fitting diagram showing the dose-effect relationship of calcipotriol when used alone in part 5.1 of Example 5.

Fig. 17 is a graph showing the effect of calcipotriol when used alone on tyrosinase activity of zebrafish in part 5.2 of Example 5.

Fig. 18 is a graph showing the effect of fluoxetine and $VD_3$ in different ratio on the melanin content of zebrafish in part 6.1 of Example 6.

Fig. 19 is a graph showing the expected additive effect and the actual effect with vitamin $D_3$ concentration as abscissa in the combined group of part 6.2 of Example 6.

Fig. 20 is a graph showing the expected additive effect and the actual effect with the concentration of calcipotriol as the abscissa in the combined group in part 7.1 of Example 7.

Fig. 21 is a graph showing the effects of fluoxetine cream, calcipotriol cream, fluoxetine cream plus calcipotriol cream, and fluoxetine calcipotriol compound cream on the back skin color of C57BL/6 mice of hydroquinone model subjected to physical depilation with rosin and paraffin in part 8.4 of Example 8.

Fig. 22 is a graph showing the effect of fluoxetine cream, calcipotriol cream, fluoxetine cream plus calcipotriol cream, and fluoxetine calcipotriol compound cream on HE staining of the skin hair follicles on the back of C57BL/6 mice of hydroquinone model subjected to physical depilation with rosin and paraffin in part 8.4 of Example 8.

**EMBODIMENTS**

Hereinafter, the specific embodiments of the present invention will be described in detail with reference to the drawings, but it should be understood that the protection scope of the present invention is not limited by the specific embodiments. Unless otherwise specified, in following Examples, fluoxetine refers to fluoxetine racemate hydrochloride and R- fluoxetine hydrochloride. In the following Examples and drawings, Flu is short for fluoxetine, $VD_3$ is short for vitamin $D_3$, Cal is short for calcipotriol and Tal is short for tacalcitol. The dose in zebrafish experiment and mice experiment in Examples is equivalent to 0.01 and 0.081 of clinical dose, that is, the dose range of fluoxetine to zebrafish in Examples is 0.1-100 $\mu$g/ml, which is equivalent to 0.01-10 mg/ml or 0.01-10 mg/g of clinical dose. Reference [12] can be reffered for specific conversion methods. C57BL/6 mice used in Examples were purchased from Experimental Animal Center of Yangzhou University; Adult zebrafish were purchased from the National Zebrafish Resource Center, and zebrafish embryos were obtained by self-incubation in the laboratory. Fluoxetine was purchased from Zhejiang Puluojiayuan Pharmaceutical Co., Ltd.; Castriol was purchased from Shanghai Yishi Chemical Co., Ltd.; Vitamin $D_3$, PTU and tacalcitol were purchased from Sigma Company.

**Example 1**

1.1 Effects of fluoxetine, vitamin $D_3$, calcipotriol and tacalcitol when used alone on survival rate of zebrafish. The embryos obtained by fertilization of zebrafish were grouped as follows:

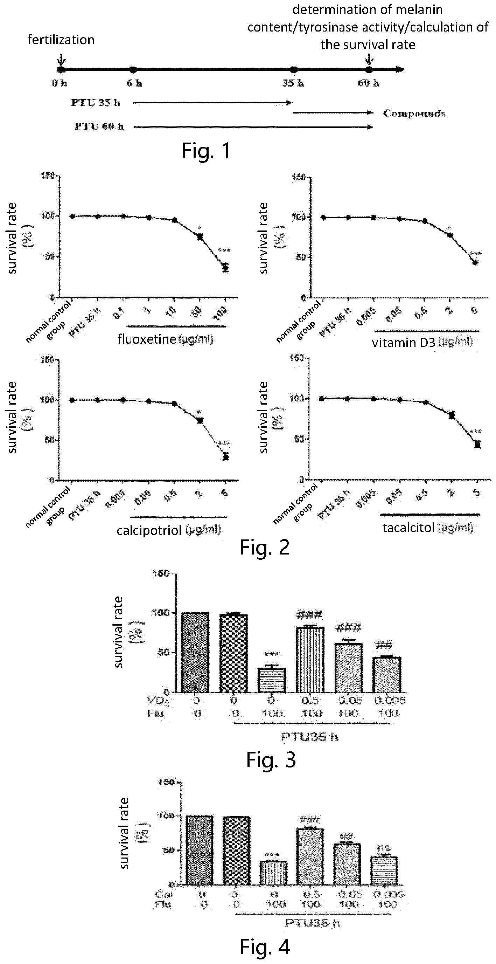

Normal control group: i.e. non-dose group;
PTU(1-phenyl-2-thiourea, PTU is a reversible tyrosinase inhibitor) 35h treatment group: the zebrafish embryos which had developed for 6 hours were put into aqueous solution containing 0.2 mM PTU and cultured until the embryos were developed for 35 hours, and then the zebrafish embryos were put in purified water for 25 hours to observe the survival rate of the zebrafish;
PTU35h + fluoxetine treatment group: the same operations as the above-mentioned PTU35h treatment group were carried out except that the pure water was replaced by aqueous solutions of fluoxetine with concentration of 0.1, 1, 10 and 100 $\mu$g/ml, respectively;
PTU35h + calcipotriol treatment group: the same operations as the above-mentioned PTU35h treatment group were carried out except that the pure water was replaced by aqueous solutions of calcipotriol with concentration of 0.005, 0.05, 0.5 and 5 $\mu$g/ml, respectively;
PTU35h + tacalcitol treatment group: the same operations as those of the above-mentioned PTU35h treatment group were carried out, except that the pure water was replaced by aqueous solutions of tacalcitol with concentration of 0.005, 0.05, 0.5 and 5 $\mu$g/ml, respectively;
PTU35h + $VD_3$ treatment group: the same operations as the above-mentioned PTU35h treatment group were carried out except that the pure water was replaced by aqueous solutions of $VD_3$ with concentration of 0.005, 0.05, 0.5 and 5 $\mu$g/ml, respectively. The results were shown in Fig. 2, wherein * means P<0.05 and *** means P<0.001 when compared with Normal Control group.

As shown in Fig. 2, $VD_3$ significantly inhibited the survival rate of zebrafish at a dose of 5 $\mu$g/ml, with $IC_{50}$ = 4.5 $\mu$ g/ml; Calcipotriol significantly inhibited the survival rate of zebrafish at the dose of 5 $\mu$g/ml, $IC_{50}$ = 3.5 $\mu$ g/ml; tacalcitol significantly inhibited the survival rate of zebrafish at a dose of 5 $\mu$g/ml, $IC_{50}$ = 4.0 $\mu$g/ml; fluoxetine had a significant effect on the survival rate of zebrafish at a dose of 100 $\mu$g/ml, $IC_{50}$=89.7 $\mu$g/ml.

**Example 2**

2.1 Effect of fluoxetine (100 $\mu$g/ml) in combination with different concentration of $VD_3$ on survival rate of zebrafish

Zebrafish embryos were grouped as follows:

Normal control group: i.e. non-dose group;
PTU35h treatment group: the same operations as those of PTU35h treatment group in Example 1 were carried out;
PTU35h + fluoxetine (100 $\mu$g/ml) treatment group: the same operations as the above-mentioned PTU35h treatment group were carried out except that the pure water was replaced by aqueous solution of fluoxetine with concentration of 100 $\mu$g/ml;
PTU35h + fluoxetine (100 $\mu$g/ml)+ $VD_3$ (0.005-0.5 $\mu$g/ml) treatment group: the same operations as the above-mentioned PTU35h + fluoxetine (100 $\mu$g/ml) treatment group were carried out except that aqueous solutions of $VD_3$ with concentration of 0.005, 0.05, 0.5$\mu$g/ml were further added to the aqueous solution of fluoxetine with concentration of 100 $\mu$g/ml, respectively.

The results were shown in Fig. 3, wherein *** means $p < 0.001$ when compared with PTU35h treatment group; ## means $P<0.01$, and ### means $P<0.001$ when compared with fluoxetine (100 $\mu$g/ml) group.

As shown in Fig. 3, fluoxetine significantly inhibited the survival rate of zebrafish at 100 $\mu$g/ml, and the mortality rate of zebrafish gradually decreased with the increase of $VD_3$ dosage. The results suggest that $VD_3$ in combination with fluoxetine can reduce the toxicity of fluoxetine to zebrafish.

2.2 Effect of fluoxetine (100 $\mu$g/ml) in combination with different concentration of calcipotriol on survival rate of zebrafish

Zebrafish embryos were grouped as follows:

Normal control group: i.e. non-dose group;
PTU35h treatment group: the same operations as those of PTU35h treatment group in Example 1 were carried out;
PTU35h + fluoxetine (100 $\mu$g/ml) treatment group: the same operations as the above-mentioned PTU35h treatment group were carried out except that the pure water was replaced by aqueous solution of fluoxetine with concentration of 100 $\mu$g/ml;
PTU35h + fluoxetine (100 $\mu$g/ml)+ calcipotriol (0.005-0.5 $\mu$g/ml) treatment group: the same operations as the above-mentioned PTU35h + fluoxetine (100 $\mu$g/ml) treatment group were carried out except that aqueous solutions of calcipotriol with concentration of 0.005, 0.05, 0.5$\mu$g/ml were further added to the aqueous solution of fluoxetine with concentration of 100 $\mu$g/ml, respectively.

The results were shown in Fig. 4, wherein *** means $p < 0.001$ when compared with PTU35h treatment group; ## means $P<0.01$, ### means $P<0.001$ and ns means $P>0.05$ when compared with fluoxetine (100 $\mu$g/ml) group.

As shown in Fig. 4, fluoxetine significantly inhibited the survival rate of zebrafish at 100 $\mu$g/ml, and the mortality rate of zebrafish gradually decreased with the increase of calcipotriol dosage. The results suggest that calcipotriol in combination with fluoxetine can reduce the toxicity of fluoxetine to zebrafish.

2.3 Effect of fluoxetine (100 $\mu$g/ml) in combination with different concentration of tacalcitol on survival rate of zebrafish

Zebrafish embryos were grouped as follows:

Normal control group: i.e. non-dose group;
PTU35h treatment group: the same operations as those of PTU35h treatment group in Example 1 were carried out;
PTU35 h+ fluoxetine (100 $\mu$g/ml) treatment group: the same operations as the above-mentioned PTU35h treatment group were carried out except that the pure water was replaced by aqueous solution of fluoxetine with concentration of 100 $\mu$g/ml;
PTU35h + fluoxetine (100 $\mu$g/ml) + tacalcitol (0.005-0.5 $\mu$g/ml) treatment group: the same operations as the above-mentioned PTU35h + fluoxetine (100 $\mu$g/ml) treatment group were carried out except that aqueous solutions of tacalcitol with concentration of 0.005, 0.05, 0.5$\mu$g/ml were further added to the aqueous solution of fluoxetine with concentration of 100 $\mu$g/ml, respectively.

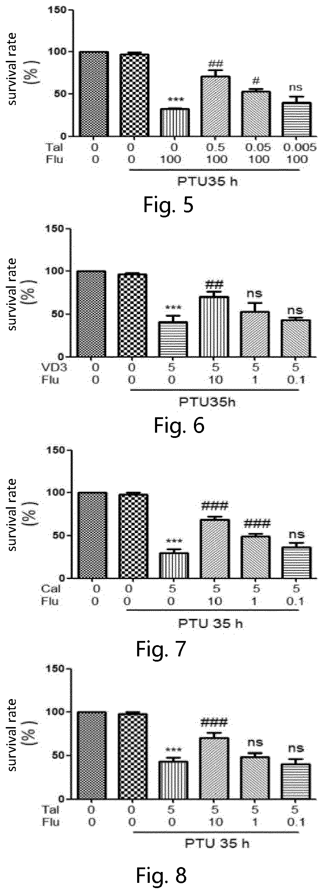

The results were shown in Fig. 5, wherein *** means $p < 0.001$ when compared with PTU35h treatment group; # means $P<0.05$, ## means $P<0.01$ and ns means $P>0.05$ when compared with fluoxetine (100 $\mu$g/ml) group.

As shown in Fig. 5, fluoxetine significantly inhibited the survival rate of zebrafish at 100 $\mu$g/ml, and the mortality rate of zebrafish gradually decreased with the increase of tacalcitol dosage. The results suggest that tacalcitol in combination with fluoxetine can reduce the toxicity of fluoxetine to zebrafish.

2.4 Effect of $VD_3$ (5 $\mu$g/ml) in combination with different concentration of fluoxetine on survival rate of zebrafish

Zebrafish embryos were grouped as follows:

Normal control group: i.e. non-dose group;
PTU35h treatment group: the same operations as those of PTU35h treatment group in Example 1 were carried out;
PTU35h + $VD_3$ (5 $\mu$g/ml) treatment group: the same operations as those of the PTU35h treatment group in Example 1 were carried out except that the pure water was replaced by aqueous solution of $VD_3$ with concentration of 5 $\mu$g/ml.

PTU35h + VD$_3$ (5 $\mu$g/ml)+ fluoxetine (0.1-10 $\mu$g/ml) treatment group: the same operations as those in the above-mentioned PTU35h + VD$_3$ (5 $\mu$g/ml) treatment group were carried out except that aqueous solutions of fluoxetine with concentration of 0.1, 1, 10$\mu$g/ml were further added to the aqueous solution of VD$_3$ with concentration of 5 $\mu$g/ml, respectively.

The results were shown in Fig. 6, wherein *** means p < 0.001 when compared with PTU35h treatment group; ## means P<0.01, and ns means *P>0.05* when compared with VD$_3$ (5 $\mu$g/ml) treatment group.

As shown in Fig. 6, VD$_3$ significantly inhibited the survival rate of zebrafish at 5 $\mu$g/ml, and the mortality rate of zebrafish gradually decreased with the increase of fluoxetine dosage. The results suggest that VD$_3$ in combination with fluoxetine can also reduce the toxicity of VD$_3$ to zebrafish.

2.5 Effect of calcipotriol (5 $\mu$g/ml) in combination with different concentration of fluoxetine on survival rate of zebrafish

Zebrafish embryos were grouped as follows:

Normal control group: i.e. non-dose group;
PTU35h treatment group: the same operations as those of PTU35h treatment group in Example 1 were carried out;
PTU35h + calcipotriol (5 $\mu$g/ml) treatment group: the same operations as those the above-mentioned PTU35h treatment group were carried out except that the pure water was replaced by aqueous solutions of calcipotriol with concentration of 5 $\mu$g/ml;
PTU35h + calcipotriol (5 $\mu$g/ml) + fluoxetine (0.1-10 $\mu$g/ml) treatment group: the same operations as those of the above-mentioned PTU35h+ calcipotriol (5 $\mu$g/ml) treatment group were carried out except that aqueous solutions of fluoxetine with concentration of 0.1, 1, 10$\mu$g/ml were further added to the aqueous solution of calcipotriol with concentration of 5 $\mu$g/ml, respectively.

The results were shown in Fig. 7, wherein *** means p < 0.001 when compared with PTU35h treatment group; ### means P<0.001, and ns means *P>0.05* when compared with calcipotriol (5 $\mu$g/ml) treatment group.

As shown in Fig. 7, calcipotriol significantly inhibited the survival rate of zebrafish at 5 $\mu$g/ml, and the mortality rate of zebrafish gradually decreased with the increase of fluoxetine dosage. The results suggest that calcipotriol in combination with fluoxetine can also reduce the toxicity of calcipotriol to zebrafish.

2.6 Effect of tacalcitol (5 $\mu$g/ml) in combination with different concentration of fluoxetine on survival rate of zebrafish

Zebrafish embryos were grouped as follows:

Normal control group: i.e. non-dose group;
PTU35h treatment group: the same operations as those of PTU35h treatment group in Example 1 were carried out;
PTU35h+ tacalcitol (5 $\mu$g/ml) treatment group: the same operations as those the above-mentioned PTU35h treatment group were carried out except that the pure water was replaced by aqueous solutions of tacalcitol with concentration of 5 $\mu$g/ml;
PTU35h+tacalcitol (5 $\mu$g/ml) + fluoxetine (0.1-10 $\mu$g/ml) treatment group: the same operations as those of the above-mentioned PTU35h + tacalcitol (5 $\mu$g/ml) treatment group were carried out except that aqueous solutions of fluoxetine with concentration of 0.1, 1, 10$\mu$g/ml were further added to the aqueous solution of tacalcitol with concentration of 5 $\mu$g/ml, respectively.

The results were shown in Fig. 8, wherein *** means p < 0.001 when compared with PTU35h treatment group; ### means P<0.001, and ns means *P>0.05* when compared with tacalcitol (5 $\mu$g/ml) treatment group.

As shown in Fig. 8, tacalcitol significantly inhibited the survival rate of zebrafish at 5 $\mu$g/ml, and the mortality rate of zebrafish gradually decreased with the increase of fluoxetine dosage. The results suggest that tacalcitol in combination with fluoxetine can also reduce the toxicity of tacalcitol to zebrafish.

**Example 3**

3.1 Effect of fluoxetine when used alone on melanin content in zebrafish

Zebrafish were grouped as follows:

Normal Control group (Control): embryos obtained after fertilization of zebrafish were cultured in purified water for 60 hours;

PTU35h treatment group: zebrafish embryos which had developed for 6 hours were added into the aqueous solution containing 0.2 mM PTU and cultured for 29 hours until the embryos was developed for 35 hours, then the embryos were placed in purified water and continued to culture for 25 hours until the embryos were developed for 60 hours;

Fluoxetine (0.1, 0.4, 1.6, 6.4, 25.6 μg/ml) treatment group: the same operations as those of the above-mentioned PTU35h treatment group were carried out except that zebrafish embryos which had developed for 35 hours were added into the aqueous solution of fluoxetine with concentration of 0.1, 0.4, 1.6, 6.4, 25.6 μg/ml, respectively, and cultured for 25 hours. The melanin of zebrafish was quantitatively analyzed by NaOH lysis method. Zebrafish in each group were collected, excess water was removed, and then 100 μl PBS was added thereto. The thus resultant mixture was ultrasonically crushed in ice bath for 1 min, then centrifuged at 4°C and 12,000 r/min for 10 min, and the supernatant was collected for protein quantification (BCA method) to calculate the total protein content. 100 μl NaOH (containing 10% DMSO) was added to the melanin precipitate in the underlayer which was placed in a water bath at 80 °C and lysed for 2 hours. The completely dissolved melanin was added into a 96-well plate at 80 μl/ well, and the absorbance at 405 nm wavelength was measured to calculate the melanin content per milligram of protein. All the experiments were repeated for 3 times, and all the data were analyzed by ANOVA, followed by Turkey test. *** means $P < 0.001$ when compared with the normal control group; # means $P<0.05$, ## means $P< 0.01$ and ns means $P>0.05$ when compared with PTU35h group.

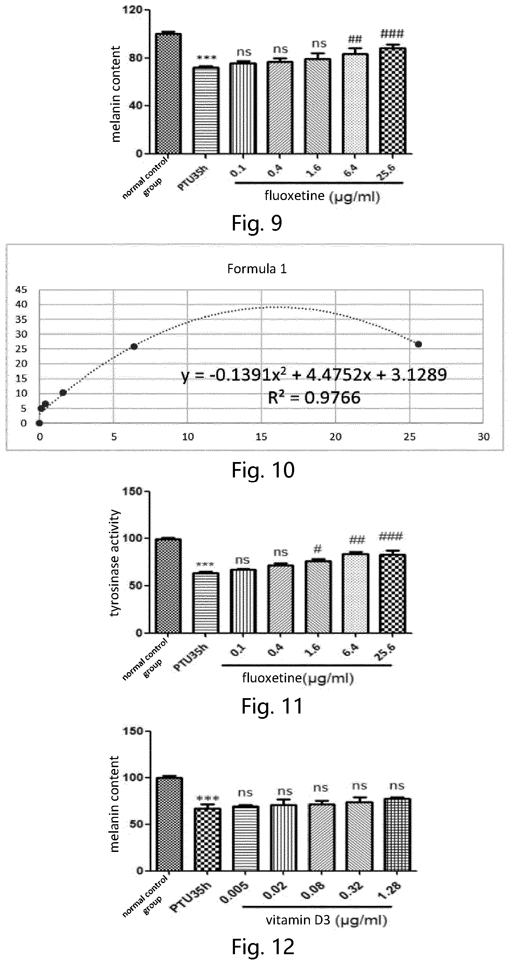

As shown in Fig. 9, when compared with the normal control group, the melanin content in the PTU35 h treatment group decreased significantly, which proved that PTU could lead to the loss of melanin in zebrafish. When compared with the PTU35h treatment group, administration of fluoxetine (6.4-25.6 μg/ml) could increase the melanin content in zebrafish in a dose-dependent manner. The calculation results of the effective rate of fluoxetine when used alone (0.1-25.6 μg/ml) on melanogenesis in zebrafish were shown in Table 1, and the polynomial fitting of the dose-effect relationship of fluoxetine was shown in Fig. 10.

Table 1. The effective rate of fluoxetine when used alone on melanogenesis in zebrafish

| Fluoxetine when used alone (μg/ml) | 0.1 | 0.4 | 1.6 | 6.4 | 25.6 |
|---|---|---|---|---|---|
| effective rate (%) | 4.962 | 6.564 | 10.260 | 25.804 | 26.536 |

$$\text{effective rate}(\%) = \left| \frac{C_{\text{administration group}} - C_{\text{blank group}}}{C_{\text{blank group}}} \right| \times 100\%;$$

$C_{\text{administration group}}$: the melanin content in administration group;

$C_{\text{blank group}}$: the melanin content of blank group

3.2 Effect of fluoxetine when used alone on tyrosinase activity of zebrafish

L-DOPA oxidation method was used to measure tyrosinase activity of zebrafish, which was as follows: supernatant obtained through centrifugation in part 3.1 in Example 3 was used to quantify protein by BCA method, and protein concentration was calculated; 10 μg protein was added into 96-well plate, PBS(0.1 M, pH 6.8) was added to 100 μl, then 100 μl of L-DOPA at 0.1% g/ml was added thereto, three wells were set for each concentration. The thus obtained mixture was incubated at 37°C in the dark for 60 min, and OD value at 475 nm was measured. All the experiments were repeated for 3 times, and all the data were analyzed by by one-way analysis of variance (ANOVA), followed by Turkey test. *** means $P < 0.001$ when compared with the normal control group; ### means $P<0.001$, ## means $P< 0.01$, # means $P < 0.05$ and ns means $P>0.05$ when compared with PTU35h treatment group.

Fig. 11 showed that the tyrosinase activity in PTU35h treatment group decreased significantly when compared with the normal control group, and fluoxetine (1.6-25.6 μg/ml) can increase the tyrosinase activity of zebrafish in a dose-dependent manner when compared with the PTU35h treatment group.

**Example 4**

4.1 Effect of VD$_3$ when used alone on melanin content in zebrafish

Zebrafish were grouped as follows:

Normal Control group (Control): embryos obtained after fertilization of zebrafish were cultured in purified water for 60 hours;

PTU35h treatment group: zebrafish embryos which had developed for 6 hours were added into the aqueous solution containing 0.2 mM PTU and cultured for 29 hours until the embryos were developed for 35 hours, then the embryos were placed in purified water and continued to culture for 25 hours until the embryos were developed for 60 hours;

$VD_3$ (0.005, 0.02, 0.08, 0.32, 1.28 $\mu$g/ml) treatment group: the same operations as those of the above-mentioned PTU35h treatment group were carried out except that zebrafish embryos which had developed for 35 hours were added into the aqueous solution of $VD_3$ with concentration of 0.005, 0.02, 0.08, 0.32, 1.28 $\mu$g/ml, respectively, and continued to culture for 25 hours.The method of measuring melanin content can refer to part 3.1 of Example 3. When compared with the normal control group, *** means P < 0.001; when compared with PTU35 h treatment group, ns means P>0.05.

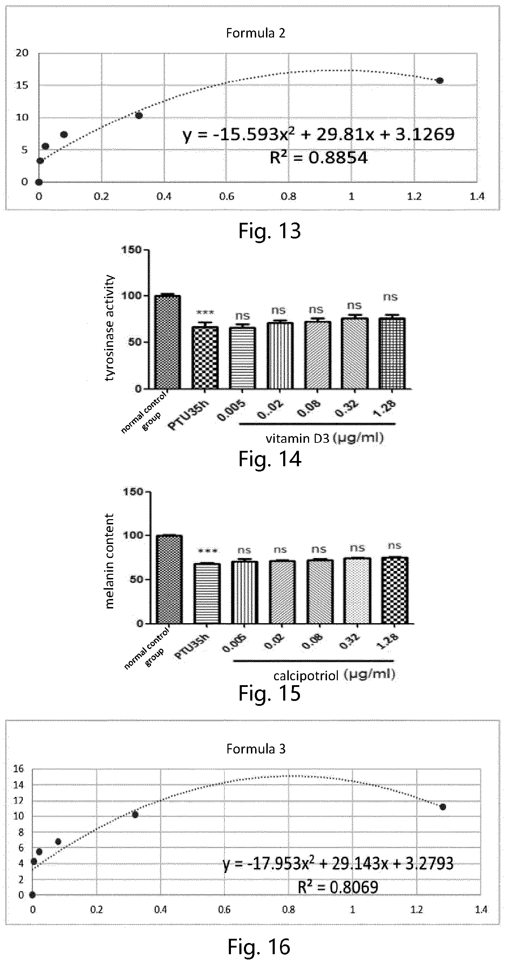

As shown in Fig. 12, when compared with the normal control group, the melanin content in the PTU35 h treatment group decreased significantly, which proved that PTU could lead to the loss of melanin in zebrafish. When compared with the PTU35h treatment group, although administration of $VD_3$ (0.005-1.28 $\mu$g/ml) can partially promote the increase of melanin content in zebrafish, there was no significant difference. The calculation results of the effective rate of $VD_3$ when used alone (0.005-1.28 $\mu$g/ml) on melanogenesis in zebrafish embryos were shown in Table 2, and the polynomial fitting of the dose-effect relationship of $VD_3$ was shown in Fig. 13.

**Table 2:** The effective rate of $VD_3$ when used alone on melanogenesis in zebrafish

| $VD_3$ when used alone ($\mu$g/ml) | 0.005 | 0.02 | 0.08 | 0.32 | 1.28 |
|---|---|---|---|---|---|
| Effective rate (%) | 3.346 | 5.564 | 7.337 | 10.315 | 15.775 |

4.2 Effect of $VD_3$ when used alone on tyrosinase activity of zebrafish

Zebrafish embryos were grouped as follows:

Normal Control group (Control): embryos obtained after fertilization of zebrafish were cultured in purified water for 60 hours;

PTU35h treatment group: zebrafish embryos which had developed for 6 hours were added into the aqueous solution containing 0.2 mM PTU and continued to culture for 29 hours until the embryos was developed for 35 hours, then the embryos were placed in purified water and continued to culture for 25 hours until the embryos were developed for 60 hours;

$VD_3$ (0.005, 0.02, 0.08, 0.32, 1.28 $\mu$g/ml) treatment group: the same operations as those of the above-mentioned PTU35h treatment group were carried out except that zebrafish embryos which had developed for 35 hours were added into the aqueous solution of $VD_3$ with concentration of 0.005, 0.02, 0.08, 0.32, 1.28 $\mu$g/ml, respectively, and cultured for 25 hours.

The method of measuring tyrosinase activity can refer to part 3.2 of Example 3. When compared with the normal control group, *** means P < 0.001; when compared with PTU35 h treatment group, ns means P>0.05.

Experimental result: Fig. 14 showed that the tyrosinase activity of zebrafish in PTU35h treatment group decreased significantly when compared with the normal control group, and $VD_3$ dose (0.005-1.28 $\mu$g/ml) had no significant effect on tyrosinase activity of zebrafish when compared with the PTU35h treatment group.

**Example 5**

5.1 Effect of calcipotriol when used alone on melanin content in zebrafish

Zebrafish embryos were grouped as follows:

Normal Control group (Control): embryos obtained after fertilization of zebrafish were cultured in purified water for 60 hours;

PTU35h treatment group: zebrafish embryos which had developed for 6 hours were added into the aqueous solution containing 0.2 mM PTU and continued to culture for 29 hours until the embryos was developed for 35 hours, then the embryos were placed in purified water and cultured for 25 hours until the embryos were developed for 60 hours;

Calcipotriol (0.005, 0.02, 0.08, 0.32, 1.28 $\mu$g/ml) treatment group: the same operations as those of the above-mentioned PTU35h treatment group were carried out except that zebrafish embryos which had developed for 35 hours were added into the aqueous solution of calcipotriol with concentration of 0.005, 0.02, 0.08, 0.32, 1.28 $\mu$g/ml, respectively, and cultured for 25 hours. The method of measuring melanin content can refer to part 3.1 of Example 3. When compared with the normal control group, *** means P < 0.001; when compared with PTU35 h treatment group, ns means P>0.05.

Experimental result: as shown in Fig. 15, when compared with the normal control group, the melanin content in the PTU35 h treatment group decreased significantly, which proved that PTU could lead to the loss of melanin in zebrafish. When compared with the PTU35h treatment group, administration of calcipotriol (0.005-1.28 $\mu$g/ml) had no significant effect on tyrosinase activity of zebrafish when compared with the PTU35h treatment group. The calculation results of the effective rate of calcipotriol when used alone (0.005-1.28 $\mu$g/ml) on melanogenesis in zebrafish were shown in Table 3, and the polynomial fitting of the dose-effect relationship of calcipotriol was shown in Fig. 16.

Table 3: The effective rate of calcipotriol when used alone on melanogenesis in zebrafish

| VD$_3$ when used alone ($\mu$g/ml) | 0.005 | 0.02 | 0.08 | 0.32 | 1.28 |
|---|---|---|---|---|---|
| Effective rate (%) | 4.296 | 5.509 | 6.773 | 10.214 | 11.197 |

5.2 Effect of calcipotriol when used alone on tyrosinase activity of zebrafish

Zebrafish were grouped as follows:

Normal Control group (Control): embryos obtained after fertilization of zebrafish were cultured in purified water for 60 hours;
PTU35h treatment group: zebrafish embryos which had developed for 6 hours were added into the aqueous solution containing 0.2 mM PTU and cultured for 29 hours until the embryos were developed for 35 hours, then the embryos were placed in purified water and continued to culture for 25 hours until the embryos were developed for 60 hours;
Calcipotriol (0.005, 0.02, 0.08, 0.32, 1.28 $\mu$g/ml) treatment group: the same operations as those of the above-mentioned PTU35h treatment group were carried out except that zebrafish embryos which had developed for 35 hours were added into the aqueous solution of calcipotriol with concentration of 0.005, 0.02, 0.08, 0.32, 1.28 $\mu$g/ml, respectively, and continued to culture for 25 hours.

The method of measuring tyrosinase activity can refer to part 3.2 of Example 3. When compared with the normal control group, *** means P < 0.001; when compared with PTU35 h treatment group, ns means P>0.05.

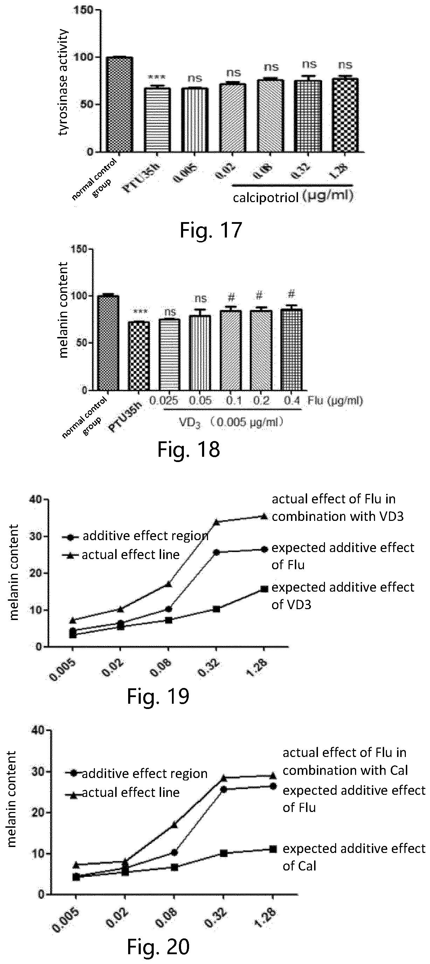

Fig. 17 showed that the tyrosinase activity of zebrafish in PTU35h treatment group decreased significantly when compared with the normal control group, and calcipotriol dose (0.005-1.28 $\mu$g/ml) had no significant effect on tyrosinase activity of zebrafish when compared with the PTU35h treatment group.

**Example 6**

6.1 Effect of fluoxetine and VD$_3$ in different ratio on melanin content in zebrafish

Zebrafish embryos were grouped as follows:

Normal Control group (Control): embryos obtained after fertilization of zebrafish were cultured in purified water for 60 hours;
PTU35h treatment group: zebrafish embryos which had developed for 6 hours were added into the aqueous solution containing 0.2 mM PTU and cultured for 29 hours until the embryos was developed for 35 hours, then the embryos were placed in purified water and cultured for 25 hours until the embryos were developed for 60 hours;
Fluoxetine + VD$_3$ (0.005 $\mu$g/ml) treatment group: the same operations as those of the above-mentioned PTU35h treatment group were carried out except that zebrafish embryos which had developed for 35 hours were added into the aqueous solution containing 0.4 $\mu$g/ml fluoxetine + 0.005 $\mu$g/ml VD$_3$, 0.2 $\mu$g/ml fluoxetine + 0.005 $\mu$g/ml VD$_3$, 0.1 $\mu$g/ml fluoxetine + 0.005 $\mu$g/ml VD$_3$, 0.05 $\mu$g/ml fluoxetine + 0.005 $\mu$g/ml VD$_3$, 0.025 $\mu$g/ml fluoxetine + 0.005 $\mu$g/ml VD$_3$, respectively, and continued to culture for 25 hours.

The method of measuring melanin content can refer to part 3.1 of Example 3. When compared with the normal control group, *** means P < 0.001; when compared with PTU35 h treatment group, ns means P>0.05 and # means P<0.05.

Fig. 18 showed that when fluoxetine and $VD_3$ were administrated to zebrafish in ratio of 5:1, 10:1, 20:1, 40:1 and 80:1, there was a significant difference at 20:1. Therefore, in the following Examples, a combination of fluoxetine and $VD_3$ in a ratio of 20:1 was studied.

6.2 Effect of fluoxetine in combination with $VD_3$ on melanin content in zebrafish

Zebrafish embryos were grouped as follows:

PTU35h treatment group: zebrafish embryos which had developed for 6 hours were added into the aqueous solution containing 0.2 mM PTU and cultured for 29 hours until the embryos was developed for 35 hours, then the embryos were placed in purified water and cultured for 25 hours until the embryos were developed for 60 hours;
Fluoxetine + $VD_3$ (0.1+0.005, 0.4+0.02, 1.6+0.08, 6.4+0.32, 25.6+1.28 $\mu$g/ml + $\mu$g/ml) treatment group: the same operations as those of the above-mentioned PTU35h treatment group were carried out except that zebrafish embryos which had developed for 35 hours were added into the aqueous solution containing 0.1 $\mu$g/ml fluoxetine +0.005 $\mu$g/ml $VD_3$, 0.4 $\mu$g/ml fluoxetine+0.02 $\mu$g/ml $VD_3$, 1.6 $\mu$g/ml fluoxetine+0.08 $\mu$g/ml $VD_3$, 6.4 $\mu$g/ml fluoxetine+0.32 $\mu$g/ml $VD_3$, 25.6 $\mu$g/ml fluoxetine+1.28 $\mu$g/ml $VD_3$, respectively, and continued to culture for 25 hours.

The method of measuring melanin content can refer to part 3.1 of Example 3.
Table 4 showed the effective rate of fluoxetine and $VD_3$ when used alone as well as fluoxetine in combination with $VD_3$ on melanogenesis in zebrafish. The expected additive effects of fluoxetine and $VD_3$ were obtained by equivalent dose conversion, as shown in Table 5 and Table 6. The expected additive effect and actual effect were plotted against the dose of $VD_3$ as the abscissa, as shown in Fig. 19. The actual additive effect when fluoxetine and vitamin D3 were used in combination was greater than the expected additive effect thereof. The results were analyzed, and shown in Table 7. Fluoxetine (0.1-25.6 $\mu$g/ml) and $VD_3$ (0.005-1.28 $\mu$g/ml) had additive effect within the dosage range.

Table 4: Dose-effect relationship data of fluoxetine (Flu) and vitamin $D_3$ ($VD_3$) when used alone and in combination

| Flu when used alone ($\mu$g/ml) | | $VD_3$ when used alone ($\mu$g/ml) | | Flu+VD3 when used in combination ($\mu$g/ml+$\mu$g/ml) | |
|---|---|---|---|---|---|
| concentration | effective rate% | concentration | effective rate% | concentration | effective rate% |
| 0.1 | 4.5 | 0.005 | 3.346 | 0.1+0.005 | 7.33 |
| 0.4 | 6.564 | 0.02 | 5.564 | 0.4+0.02 | 10.247 |
| 1.6 | 10.26 | 0.08 | 7.337 | 1.6+0.08 | 17.155 |
| 6.4 | 25.804 | 0.32 | 10.315 | 6.4+0.32 | 33.864 |
| 25.6 | 26.536 | 1.28 | 15.775 | 25.6+1.28 | 35.464 |

Table 5: Calculation table of expected additive effect by sequential equivalent dose conversion of fluoxetine (Flu)+ vitamin $D_3$($VD_3$) used in a fixed ratio using fluoxetine as the target drug.

| Dose | | equivalent dose conversion using fluoxetine as the target drug $f$(Flu+Flu$_{tx}$) | | | |
|---|---|---|---|---|---|
| Flu ($\mu$g/ml) | $VD_3$ ($\mu$g/ml) | efficacy of $VD_3$ (%) | equivalent dose of Flu (Flu$_{tx}$) | Combination of equivalent dose of Flu (Flu+Flu$_{tx}$, $\mu$g/ml) | Expected additive effect of $f$ (Flu+Flu$_{tx}$)(%) |
| (1) | (2) | (3) | (4) | (5) | (6) |
| 0.1 | 0.005 | 3.28 | 0.033 | 0.133 | 3.721 |
| 0.4 | 0.02 | 3.72 | 0.132 | 0.532 | 5.470 |
| 1.6 | 0.08 | 5.41 | 0.519 | 2.119 | 11.985 |
| 6.4 | 0.32 | 11.07 | 1.885 | 8.285 | 30.657 |
| 25.6 | 1.28 | 15.74 | 3.120 | 28.720 | 16.923 |

Note: column (3) was calculated from column (2) using Formula 2; column (4) was calculated from column (3) using Formula 1; column (5) was equal to Column (1) + Column (4); column (6) was calculated from column (5) using Formula 1.

Table 6: Calculation table of expected additive effect by sequential equivalent dose conversion of fluoxetine (Flu)+ $VD_3$ used in a fixed ratio using $VD_3$ as the target drug.

| Dose | | | equivalent dose conversion $g$(VD3+VD3$_{nx}$) using $VD_3$ as the target drug | | |
|---|---|---|---|---|---|
| Flu (μg/ml) | VD3 (μg/ml) | efficacy of Flu (%) | equivalent dose of VD3 (Cal$_{tx}$) | Combination of equivalent dose of VD3 (VD3+VD3$_{nx}$, μg/ml) | Expected additive effect of $g$ (VD3+VD3$_{nx}$) (%) |
| (1) | (2) | (3) | (4) | (5) | (6) |
| 0.1 | 0.005 | 3.151 | 0.0008 | 0.0058 | 3.299 |
| 0.4 | 0.02 | 3.218 | 0.0031 | 0.0231 | 3.806 |
| 1.6 | 0.08 | 3.486 | 0.0121 | 0.0921 | 5.741 |
| 6.4 | 0.32 | 4.547 | 0.0489 | 0.3689 | 12.002 |
| 25.6 | 1.28 | 8.629 | 0.2070 | 1.4870 | 12.976 |

Note: column (3) was calculated from column (2) using Formula 1 (see Fig. 10); column (4) was calculated from column (3) using Formula 2 (see Fig. 13); column (5) was equal to Column (2) + Column (4); column (6) was calculated from column (5) using Formula 2 (see Fig. 13).

Table 7: Summary and calculation of combination index of expected additive effect value by sequential equivalent dose conversion of fluoxetine (Flu)+ vitamin $D_3$(VD$_3$) used in a fixed ratio

| Dose | | expected additive effect value of $f$(Flu+Flu$_{tx}$) and $g$(VD$_3$+ VD$_{3nx}$) after equivalent dose conversion | | | | | |
|---|---|---|---|---|---|---|---|
| Flu (μg/ml) | VD$_3$ (μg/ml) | Expected additive effect of $f$(Flu+Flu$_{tx}$) (%) | Expected additive effect of $g$(VD$_3$+ VD$_{3nx}$)(%) | Actual effect (%) | CI$_{d1}$ | CI$_{d2}$ | Judgment |
| (1) | (2) | (3) | (4) | (5) | (6) | (7) | (8) |
| 0.1 | 0.005 | 3.721 | 3.299 | 7.330 | 1.97>1 | 2.22>1 | synergy |
| 0.4 | 0.02 | 5.470 | 3.806 | 10.247 | 1.87>1 | 2.69 >1 | synergy |
| 1.6 | 0.08 | 11.985 | 5.741 | 17.155 | 1.43>1 | 3.07>1 | synergy |
| 6.4 | 0.32 | 30.657 | 12.002 | 33.864 | 1.10>1 | 2.92>1 | synergy |
| 25.6 | 1.28 | 16.923 | 12.976 | 35.464 | 2.10>1 | 2.46>1 | synergy |

Description of data in each column: Column(3): from Table 5; Column (4): from Table 6; Column (5): from Table 4; column (6): column (5) /column (3); column (7): column (5) /column (4); Reference 13 can be referred for the standard for column (8).

**Example 7**

7.1 Effect of fluoxetine in combination with calcipotriol on melanin content in zebrafish

Zebrafish were grouped as follows:

PTU35h treatment group: zebrafish embryos which had developed for 6 hours were added into the aqueous solution containing 0.2 mM PTU and continued to culture for 29 hours until the embryos was developed for 35 hours, then the embryos were placed in purified water and cultured for 25 hours until the embryos were developed for 60 hours; Fluoxetine + calcipotriol (0.1+0.005, 0.4+0.02, 1.6+0.08, 6.4+0.32, 25.6+1.28 μg/ml + μg/ml) treatment group: the same operations as those of the above-mentioned PTU35h treatment group were carried out except that zebrafish embryos which had developed for 35 hours were added into the aqueous solution containing 0.1 μg/ml fluoxetine +0.005 μg/ml calcipotriol, 0.4 μg/ml fluoxetine+0.02 μg/ml calcipotriol, 1.6 μg/ml fluoxetine+0.08 μg/ml calcipotriol, 6.4 μg/ml fluoxetine+0.32 μg/ml calcipotriol, 25.6 μg/ml fluoxetine+1.28 μg/ml calcipotriol, respectively, and continued to culture for 25 hours.

The method of measuring melanin content can refer to part 3.1 of Example 3.

Table 8 showed the effective rate of fluoxetine and calcipotriol when used alone as well as fluoxetine in combination with calcipotriol on melanogenesis in zebrafish. The expected additive effects of fluoxetine and calcipotriol were obtained by equivalent dose conversion, as shown in Table 9 and Table 10. The expected additive effect and actual effect were plotted against the dose of calcipotriol as the abscissa, as shown in Fig. 20. The actual additive effect when fluoxetine and calcipotriol were used in combination was greater than the expected additive effect thereof. The results were analyzed, and shown in Table 11. Fluoxetine (0.1-25.6 $\mu$g/ml) and calcipotriol (0.005-1.28 $\mu$g/ml) had additive effect within the dosage range.

Table 8: Dose-effect relationship data of fluoxetine (Flu) and calcipotriol (Cal) when used alone and in combination

| Flu when used alone ($\mu$g/ml) | | Cal when used alone ($\mu$g/ml) | | Flu+Cal when used in combination ($\mu$g/ml+$\mu$g/ml) | |
|---|---|---|---|---|---|
| concentration | effective rate% | concentration | effective rate% | concentration | effective rate% |
| 0.1 | 4.5 | 0.005 | 4.296 | 0.1+0.005 | 7.33 |
| 0.4 | 6.564 | 0.02 | 5.509 | 0.4+0.02 | 8.113 |
| 1.6 | 10.26 | 0.08 | 6.773 | 1.6+0.08 | 17.155 |
| 6.4 | 25.804 | 0.32 | 10.214 | 6.4+0.32 | 28.529 |
| 25.6 | 26.536 | 1.28 | 11.197 | 25.6+1.28 | 29.063 |

Table 9: Calculation table of expected additive effect by sequential equivalent dose conversion of fluoxetine (Flu)+ calcipotriol (Cal) used in a fixed ratio using fluoxetine as the target drug.

| Dose | | equivalent dose conversion using fluoxetine as the target drug $f(Flu+Flu_{tx})$ | | | |
|---|---|---|---|---|---|
| Flu ($\mu$g/ml) | Cal ($\mu$g/ml) | efficacy of Cal (%) | equivalent dose of Flu ($Flu_{tx}$) | ($Flu+Flu_{tx}$, $\mu$g/ml) | Expected additive effect of $f(Flu+Flu_{tx})$(%) |
| (1) | (2) | (3) | (4) | (5) | (6) |
| 0.1 | 0.005 | 3.42 | 0.0005 | 0.1005 | 3.577 |
| 0.4 | 0.02 | 3.85 | 0.0013 | 0.4013 | 4.902 |
| 1.6 | 0.08 | 5.50 | 0.0042 | 1.6042 | 9.950 |
| 6.4 | 0.32 | 10.77 | 0.0140 | 6.4140 | 26.110 |
| 25.6 | 1.28 | 11.17 | 0.0148 | 25.6148 | 26.494 |

Note: column (3) was calculated from column (2) using Formula 3 (see Fig. 16); column (4) was calculated from column (3) using Formula 1 (see Fig. 10); column (5) was equal to Column (1) + Column (4); column (6) was calculated from column (5) using Formula 1 (see Fig. 10).

Table 10: Calculation table of expected additive effect by sequential equivalent dose conversion of fluoxetine (Flu)+ calcipotriol (Cal) used in a fixed ratio using calcipotriol as the target drug.

| Dose | | equivalent dose conversion $g(Cal+Cal_{nx})$ using Cal as the target drug $g$ (VD3+VD3nx) | | | |
|---|---|---|---|---|---|
| Flu ($\mu$g/ml) | Cal ($\mu$g/ml) | efficacy of Flu (%) | equivalent dose of Cal ($Cal_{tx}$) | ($Cal+Cal_{nx}$, $\mu$g/ml) | Expected additive effect of $g(Cal+Cal_{nx})$ (%) |
| (1) | (2) | (3) | (4) | (5) | (6) |
| 0.1 | 0.005 | 3.151 | 0.003 | 0.008 | 3.510 |
| 0.4 | 0.02 | 3.218 | 0.001 | 0.021 | 3.895 |
| 1.6 | 0.08 | 3.486 | 0.005 | 0.085 | 5.621 |
| 6.4 | 0.32 | 4.547 | 0.030 | 0.350 | 11.280 |

(continued)

| Dose | | equivalent dose conversion $g$(Cal+Cal$_{nx}$) using Cal as the target drug $g$ (VD3+VD3nx) | | | |
|---|---|---|---|---|---|
| Flu ($\mu$g/ml) | Cal ($\mu$g/ml) | efficacy of Flu (%) | equivalent dose of Cal (Cal$_{tx}$) | (Cal+Cal$_{nx}$, $\mu$g/ml) | Expected additive effect of $g$(Cal+Cal$_{nx}$) (%) |
| (1) | (2) | (3) | (4) | (5) | (6) |
| 25.6 | 1.28 | 8.629 | 0.142 | 1.422 | 8.427 |

Note: column (3) was calculated from column (2) using Formula 1 (see Fig. 10); column (4) was calculated from column (3) using Formula 3 (see Fig. 16); column (5) was equal to Column (1) + Column (4); column (6) was calculated from column (5) using Formula 3 (see Fig. 16).

Table 11: Summary and calculation of combination index of expected additive effect value by sequential equivalent dose conversion of fluoxetine (Flu)+ calcipotriol (Cal) used in a fixed ratio

| Dose | | expected additive effect value $f$(Flu+Flu$_{tX}$) and $g$(Cal+Cal$_{nx}$) after equivalent dose conversion | | | | | |
|---|---|---|---|---|---|---|---|
| Flu ($\mu$g/ml) | Cal ($\mu$g/ml) | Expected additive effect of $f$(Flu+Flu$_{tx}$) (%) | Expected additive effect of $g$(Cal+Cal$_{nx}$) (%) | Actual effect (%) | CI$_{d1}$ | CI$_{d2}$ | Judgment |
| (1) | (2) | (3) | (4) | (5) | (6) | (7) | (8) |
| 0.1 | 0.005 | 3.577 | 3.510 | 7.33 | 2.05>1 | 2.09>1 | synergy |
| 0.4 | 0.02 | 4.902 | 3.895 | 8.113 | 1.65>1 | 2.08>1 | synergy |
| 1.6 | 0.08 | 9.950 | 5.621 | 17.155 | 1.72>1 | 3.05>1 | synergy |
| 6.4 | 0.32 | 26.110 | 11.280 | 28.529 | 1.09>1 | 2.53>1 | synergy |
| 25.6 | 1.28 | 26.494 | 8.427 | 29.063 | 1.10>1 | 3.45>1 | synergy |

Description of data in each column: Column(3) came from Table 9; Column (4) came from Table 10; Column (5) came from Table 8; column (6) equals to column (5)/column (3); column (7) equals to column (5) /column (4); Reference 13 can be referred for the standard for column (8).

**Example 8**

8.1. Preparation of compound cream containing fluoxetine and calcipotriol

| | | |
|---|---|---|
| fluoxetine | 0.1% | (g/g) |
| Calcipotriol | 0.005% | (g/g) |
| DL-$\alpha$-tocopherol | 0.03% | (g/g) |
| stearic acid | 2.5% | (g/g) |
| octadecanol | 10.5% | (g/g) |
| SDS | 0.5% | (g/g) |
| glycerin | 10% | (g/g) |
| propanediol | 3% | (g/g) |
| liquid paraffin | 8% | (g/g) |
| Purified water | 65.365% | (g/g) |

The above components were all in percentage by weight. Firstly, octadecanol, stearic acid and liquid paraffin were heated to 80°C or above, and kept under the temperature so as to melt them into liquid as solution I; secondly, SDS, glycerin, propylene glycol, tocopherol and purified water were heated to 80°C or above and the thus obtained mixture was used as solution II. Then fluoxetine and calcipotriol screened by 200 meshes were added into solution I in sequence, the thus obtained mixture was emulsified under vacuum stirring for 30 minutes, then further emulsified while stirring and cooling. When the temperature was reduced to about 35°C, the thus obtained mixture was filled into packaging materials.

8.2 Preparation of cream containing 0.1% (g/g) of fluoxetine

| | | |
|---|---|---|
| fluoxetine | 0.1% | (g/g) |
| DL-$\alpha$-tocopherol | 0.03% | (g/g) |
| stearic acid | 2.5% | (g/g) |
| octadecanol | 10.5% | (g/g) |
| SDS | 0.5% | (g/g) |
| glycerin | 10% | (g/g) |
| propanediol | 3% | (g/g) |
| liquid paraffin | 8% | (g/g) |
| Purified water | 65.370% | (g/g) |

The above components were all in percentage by weight. Firstly, octadecanol, stearic acid and liquid paraffin were heated to 80 °C or above, and kept under the temperature so as to melt them into liquid as solution I; secondly, SDS, glycerin, propylene glycol, tocopherol and purified waterwere heated to 80°C or above and the thus obtained mixture was used as solution II; Then fluoxetine screened by 200 meshes was added into solution I, the thus obtained mixture was emulsified under vacuum stirring for 30 minutes, then further emulsified while stirring and cooling. When the temperature was reduced to about 35°C, the thus obtained mixture was filled into packaging materials.

8.3 Preparation of cream containing 0.005% of calcipotriol

| | | |
|---|---|---|
| calcipotriol | 0.005% | (g/g) |
| DL-$\alpha$-tocopherol | 0.03% | (g/g) |
| stearic acid | 2.5% | (g/g) |
| octadecanol | 10.5% | (g/g) |
| SDS | 0.5% | (g/g) |
| glycerin | 10% | (g/g) |
| propanediol | 3% | (g/g) |
| liquid paraffin | 8% | (g/g) |
| Purified water | 65.465% | (g/g) |

The above components were all in percentage by weight. Firstly, octadecanol, stearic acid and liquid paraffin were heated to 80 °C or above, and kept under the temperature so as to melt them into liquid as solution I; secondly, SDS, glycerin, propylene glycol, tocopherol and purified waterwere heated to 80°C or above and the thus obtained mixture was used as solution II. Then calcipotriol screened by 200 meshes was added into solution I, the thus obtained mixture was emulsified under vacuum stirring for 30 minutes, then further emulsified while stirring and cooling. When the temperature was reduced to about 35°C, the thus obtained mixture was filled into packaging materials.

8.4 Effect of compound cream containing fluoxetine and calcipotriol on melanogenesis in C57BL/6 mice

The above-mentioned cream containing 0.1% (g/g) of fluoxetine and the cream containing 0.005% (g/g) of calcipotriol and the compound cream containing 0.1% (g/g) of fluoxetine and 0.005% (g/g) of calcipotriol were selected to study their effects on melanogenesis in C57BL/6 mice of hydroquinone model.

70 healthy C57BL/6 male mice aged 6-8 weeks were fed adaptively for one week, and were randomly grouped as (1) normal control group (control group), (2) hydroquinone model group (model group), (3) blank matrix group (matrix group), (4) group of cream containing 0.1% (g/g) of fluoxetine, (5) group of the cream containing 0.005% (g/g) of calcipotriol; (6) group of administration with the cream containing 0.1% (g/g) of fluoxetine and the cream containing 0.005% (g/g) of calcipotriol at different time; 7) group of compound cream containing 0.1% (g/g) of fluoxetine and 0.005% (g/g) of calcipotriol, (8) methoxysarin group (purchased from Chongqing Huabang Pharmaceutical Co., Ltd.). The dosage of each ointment was 16.35 mg/cm$^2$. After the back of the mice was unhaired with rosin paraffin, (1) the group (1) (the normal control group) was not treated; (2) after the group (2) (model group) was coated with 2.5% hydroquinone gel in the morning for 10 days, the group (2) was only administrated with 2.5% hydroquinone; (3) the group (3) (matrix group) was smeared with hydroquinone in the morning and blank matrix in the afternoon; (4) the group (4) was smeared with the cream containing 0.1% (g/g) of fluoxetine in the afternoon; (5) the group (5) was smeared with the cream containing 0.005% (g/g) of calcipotriol in the afternoon; (6) the group (6) was smeared with 0.1% g/g fluoxetine, and then smeared with the cream containing 0.005% (g/g) of calcipotriol 12 hours later; (7) the group (7) was smeared with the compound cream containing 0.1% (g/g) of fluoxetine and 0.005% (g/g) of calcipotriol at a dose of 16.25 mg/cm$^2$; (8) the group (8) (Methoxy-sarin group) was smeared with 0.1 ml of Methoxy-sarin. After depilation, the back skin of C57BL/6 mice was photographed and observed every day. After depilation, the skin on back of C57BL/6 mice was photographed and observed every day. After 30 days of administration, the mice were sacrificed by cervical dislocation. The skin on the administration site of the back was taken and fixed in 4% paraformaldehyde for HE sectioning.

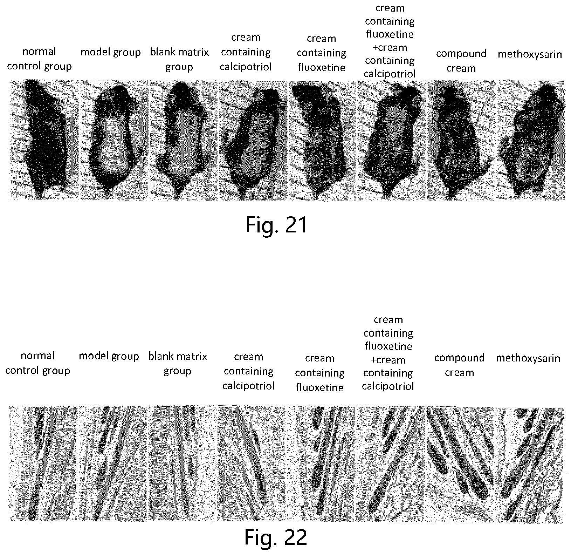

The results of appearance observation and HE staining were shown in Fig. 21 and Fig. 22. When compared with the normal control group, the skin color on the back of hydroquinone model group decreased significantly, and melanin in hair follicle decreased. The matrix group had similar case to the model group. Compared with the matrix group, the skin color of the back in the group of cream containing fluoxetine was significantly darker, and melanin in hair follicles increased. The skin color of the back in the group of the cream containing calcipotriol also deepened to some extent, and melanin in hair follicle increased compared with matrix group. When the cream containing fluoxetine and the cream containing calcipotriol were administrated at different time, the skin color and hair follicle melanin increased, but the change of skin color and hair follicle melanin was not obvious when compared with the group in which the cream containing fluoxetine was used alone and the group in which the cream containing calcipotriol was used alone. The skin color of the back and hair follicle melanin in the group of the compound cream containing fluoxetine and calcipotriol were significantly stronger than the group in which the cream containing fluoxetine was used alone and the group in which the cream containing calcipotriol was used alone, and the effect was also stronger than that of the group in which the cream containing fluoxetine and the cream containing calcipotriol were used at different time.

References:

[1] Wang Xiaoyan, Wang Tinglin, Zhou Cheng, et al. Epidemiological survey of vitiligo in six provinces and cities in China [J]. Chinese Journal of Dermatology, 2010, 43(7):463-466.

[2] Liao S, Shang J, Tian X, et al. Up-regulation of melanin synthesis by the antidepressant fluoxetine[J]. Experimental Dermatology, 2012, 21(8):635-637.

[3] Zhou L, Cai M, Ren Y, et al. The different roles of 5-HT1A/2A receptors in fluoxetine ameliorated pigmentation of C57BL/6 mouse skin in response to stress[J]. Journal of Dermatological Science, 2018, 92(3):222-229.

[4] Gu Wentao, Xu Hui, Ma Hong, et al. Detection of serum 25- hydroxyvitamin D level in patients with vitiligo and observation of therapeutic effect of calcipotriol [J]. Journal of Clinical Dermatology, 2016(8):572-574.

[5] Song Wenting, Zhao Guang. Research progress of vitiligo and autoimmune diseases [J]. Chinese Journal of Aesthetic Medicine, 2011,20(2):327-330.

[6] Gong Q, Li X, Sun J, et al. The effects of calcipotriol on the dendritic morphology of human melanocytes under oxidative stress and a possible mechanism: Is it a mitochondrial protector?[J]. Journal of Dermatological Science, 2015, 77(2):117-124.

[7] zhangqian. Systematic evaluation of tacalcitol and calcipotriol ointment in the treatment of mild to moderate vitiligo [J]. Forum of Primary Medicine, 2018(14).

[8] Gu Wentao, Xu Hui, Ma Hong, et al. Detection of serum 25- hydroxyvitamin D level in patients with vitiligo and observation of therapeutic effect of calcipotriol [J]. Journal of Clinical Dermatology, 2016(8):572-574.

[9] Tang Luyan, Fu Wenwen, Zhang Yong, et al. Effect of calcipotriol on melanogenesis in melanocytes [J]. Chinese Journal of Dermatology, 2009, 42(11):771-773.

[10] Khullar G, Kanwar A J, Singh S, et al. Comparison of efficacy and safety profile of topical calcipotriol ointment in combination with NB - UVB vs. NB - UVB alone in the treatment of vitiligo: a 24-week prospective right-left comparative clinical trial[J]. Journal of the European Academy of Dermatology and Venereology, 2014, 29(5):925-932.

[11] Coulter D M, Pillans P I. Fluoxetine and extrapyramidal side effects.[J]. American Journal of Psychiatry, 1989, 146(10):1352-3.

[12] Huang Jihan, Huang Xiaohui, Chen Zhiyang, et al. Equivalent dose conversion between animals and between animals and humans in pharmacological experiments [J]. chinese journal of clinical pharmacology and therapeutics, 2004, 9(9):1069-1072.

[13] Yuan Shoujun. Mathematical law of efficacy addition of multi-drug combination and quantitative calculation method of synergistic antagonism [M]. Jiangsu Phoenix Science and Technology Press, 2016, the first edition.

[14] Parsad Davinder, Kanwar AJ. Topical vitamin D analogues in the treatment of vitiligo [J]. Pigment Cell and Melanoma Research, 2009, 22(4): 487-488.

[15] Ameen M, Exarchou V, Chu AC. Topical calcipotriol as monotherapy and in combination with psoralen plus ultraviolet A in the treatment of vitiligo [J]. British Journal of Dermatology, 2008, 145(3): 476-479.

**Claims**

1. A preparation comprising a composition comprising fluoxetine and vitamin D3 or derivatives of vitamin $D_3$, wherein vitamin D3 or its derivatives is one or more selected from the group consisting of vitamin D3, calcipotriol, tacalcitol and calcitriol, and the preparation is a cream, an_ointment, a gel or a paste.

2. The preparation according to claim 1, wherein fluoxetine is selected from the group consisting of fluoxetine racemate, R-fluoxetine or their pharmaceutically acceptable salts.

3. The preparation according to claim 1, wherein the weight ratio of fluoxetine to vitamin D3 or its derivatives is 10 or more, preferably 20 or more.

4. The preparation according to claim 1, wherein the weight ratio of fluoxetine to vitamin D3 or its derivatives is 500 or less, preferably 400 or less, more preferably 200 or less.

5. The preparation according to any one of claims 1-4, wherein the preparation further comprises pharmaceutically acceptable excipients.

6. The preparation according to any one of claims 1 to 5, wherein the composition is a composition for use in the treatment and/or alleviation of depigmentation disorder.

7. The preparation for use according to claim 6, wherein the depigmentation disorder is selected from the group consisting of vitiligo, pityriasis alba, nevus anemicus and albinism.

8. The preparation according to any one of claims 1 to 5, wherein the composition is used for treating white hair.

**Patentansprüche**

1. Zubereitung, aufweisend eine Zusammensetzung, die Fluoxetin und Vitamin D3 oder Derivate von Vitamin D3 aufweist, wobei Vitamin D3 oder seine Derivate eines oder mehrere sind, ausgewählt aus der Gruppe bestehend aus Vitamin D3, Calcipotriol, Tacalcitol und Calcitriol, und die Zubereitung eine Creme, eine Salbe, ein Gel oder eine Paste ist.

2. Die Zubereitung gemäß Anspruch 1, wobei Fluoxetin aus der Gruppe bestehend aus Fluoxetin-Racemat, R-Fluoxetin oder deren pharmazeutisch verträglichen Salzen ausgewählt ist.

3. Die Zubereitung gemäß Anspruch 1, wobei das Gewichtsverhältnis von Fluoxetin zu Vitamin D3 oder dessen Derivaten 10 oder mehr, vorzugsweise 20 oder mehr, beträgt.

4. Die Zubereitung gemäß Anspruch 1, wobei das Gewichtsverhältnis von Fluoxetin zu Vitamin D3 oder seinen Derivaten 500 oder weniger, vorzugsweise 400 oder weniger, stärker bevorzugt 200 oder weniger, beträgt.

5. Die Zubereitung gemäß einem der Ansprüche 1 bis 4, wobei die Zubereitung ferner pharmazeutisch verträgliche Hilfsstoffe aufweist.

6. Die Zubereitung gemäß einem der Ansprüche 1 bis 5, wobei die Zusammensetzung eine Zusammensetzung zur Verwendung bei der Behandlung und/oder Linderung einer Depigmentierungsstörung ist.

**7.** Die Zubereitung zur Verwendung gemäß Anspruch 6, wobei die Depigmentierungsstörung ausgewählt ist aus der Gruppe bestehend aus Vitiligo, Pityriasis alba, Naevus anaemicus und Albinismus.

**8.** Die Zubereitung gemäß einem der Ansprüche 1 bis 5, wobei die Zusammensetzung zur Behandlung von weißem Haar verwendet wird.

**Revendications**

**1.** Préparation comprenant une composition comprenant de la fluoxétine et de la vitamine D3 ou des dérivés de vitamine $D_3$,
dans laquelle la vitamine D3 ou ses dérivés sont un ou plusieurs choisis dans le groupe constitué de la vitamine D3, le calcipotriol, le tacalcitol et le calcitriol, et la préparation est une crème, un onguent, un gel ou une pâte.

**2.** Préparation selon la revendication 1, dans laquelle la fluoxétine est choisie dans le groupe constitué du racémique de fluoxétine, du R-fluoxétine ou leurs sels pharmaceutiquement acceptables.

**3.** Préparation selon la revendication 1, dans laquelle le rapport pondéral de la fluoxétine sur la vitamine D3 ou ses dérivés est de 10 ou plus, de préférence de 20 ou plus.

**4.** Préparation selon la revendication 1, dans laquelle le rapport pondéral de la fluoxétine sur la vitamine D3 ou ses dérivés est de 500 ou moins, de préférence de 400 ou moins, plus préférablement de 200 ou moins.

**5.** Préparation selon l'une quelconque des revendications 1 à 4, dans laquelle la préparation comprend en outre des excipients pharmaceutiquement acceptables.

**6.** Préparation selon l'une quelconque des revendications 1 à 5, dans laquelle la composition est une composition pour une utilisation dans le traitement et/ou l'atténuation d'un trouble de dépigmentation.

**7.** Préparation pour utilisation selon la revendication 6, dans laquelle le trouble de dépigmentation est choisi dans le groupe constitué du vitiligo, du pityriasis alba, du naevus anemicus et de l'albinisme.

**8.** Préparation selon l'une quelconque des revendications 1 à 5, dans laquelle la composition est utilisée pour le traitement des cheveux blancs.

Fig. 1

Fig. 2

Fig. 3

Fig. 4

Fig. 5

Fig. 6

Fig. 7

Fig. 8

Fig. 9

Fig. 10

Fig. 11

Fig. 12

**Formula 2**

$$y = -15.593x^2 + 29.81x + 3.1269$$
$$R^2 = 0.8854$$

Fig. 13

Fig. 14

Fig. 15

**Formula 3**

$$y = -17.953x^2 + 29.143x + 3.2793$$
$$R^2 = 0.8069$$

Fig. 16

Fig. 17

Fig. 18

Fig. 19

Fig. 20

24

Fig. 21

Fig. 22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN ZL201110403173
- CA 2877423 A
- US 9833424 B2
- CN 107375428 A

**Non-patent literature cited in the description**

- the consensus of diagnosis and treatment of vitiligo. 2018
- **WANG XIAOYAN ; WANG TINGLIN ; ZHOU CHENG et al.** Epidemiological survey of vitiligo in six provinces and cities in China [J. *Chinese Journal of Dermatology,* 2010, vol. 43 (7), 463-466
- **LIAO S ; SHANG J ; TIAN X et al.** Up-regulation of melanin synthesis by the antidepressant fluoxetine[J. *Experimental Dermatology,* 2012, vol. 21 (8), 635-637
- **ZHOU L ; CAI M ; REN Y et al.** The different roles of 5-HT1A/2A receptors in fluoxetine ameliorated pigmentation of C57BL/6 mouse skin in response to stress[J. *Journal of Dermatological Science,* 2018, vol. 92 (3), 222-229
- **GU WENTAO ; XU HUI ; MA HONG et al.** Detection of serum 25- hydroxyvitamin D level in patients with vitiligo and observation of therapeutic effect of calcipotriol [J. *Journal of Clinical Dermatology,* 2016, (8), 572-574
- **SONG WENTING ; ZHAO GUANG.** Research progress of vitiligo and autoimmune diseases [J. *Chinese Journal of Aesthetic Medicine,* 2011, vol. 20 (2), 327-330
- **GONG Q ; LI X ; SUN J et al.** The effects of calcipotriol on the dendritic morphology of human melanocytes under oxidative stress and a possible mechanism: Is it a mitochondrial protector?[J. *Journal of Dermatological Science,* 2015, vol. 77 (2), 117-124
- **ZHANGQIAN.** Systematic evaluation of tacalcitol and calcipotriol ointment in the treatment of mild to moderate vitiligo [J. *Forum of Primary Medicine,* 2018, (14
- **TANG LUYAN ; FU WENWEN ; ZHANG YONG et al.** Effect of calcipotriol on melanogenesis in melanocytes [J. *Chinese Journal of Dermatology,* 2009, vol. 42 (11), 771-773
- **KHULLAR G ; KANWAR A J ; SINGH S et al.** Comparison of efficacy and safety profile of topical calcipotriol ointment in combination with NB - UVB vs. NB - UVB alone in the treatment of vitiligo: a 24-week prospective right-left comparative clinical trial[J. *Journal of the European Academy of Dermatology and Venereology,* 2014, vol. 29 (5), 925-932
- **COULTER D M ; PILLANS P I.** Fluoxetine and extrapyramidal side effects.[J. *American Journal of Psychiatry,* 1989, vol. 146 (10), 1352-3
- **HUANG JIHAN ; HUANG XIAOHUI ; CHEN ZHIYANG et al.** Equivalent dose conversion between animals and between animals and humans in pharmacological experiments [J. *chinese journal of clinical pharmacology and therapeutics,* 2004, vol. 9 (9), 1069-1072
- **YUAN SHOUJUN.** Mathematical law of efficacy addition of multi-drug combination and quantitative calculation method of synergistic antagonism [M. Jiangsu Phoenix Science and Technology Press, 2016
- **PARSAD DAVINDER ; KANWAR AJ.** Topical vitamin D analogues in the treatment of vitiligo [J. *Pigment Cell and Melanoma Research,* 2009, vol. 22 (4), 487-488
- **AMEEN M ; EXARCHOU V ; CHU AC.** Topical calcipotriol as monotherapy and in combination with psoralen plus ultraviolet A in the treatment of vitiligo [J. *British Journal of Dermatology,* 2008, vol. 145 (3), 476-479